# EUROPEAN PATENT APPLICATION

(11) **EP 0 850 570 A2**
(43) Date of publication of application: **01.07.1998**
(21) Application number: 97307843.9
(22) Date of filing: 03.10.1997
(51) Int. Cl.: A23L 1/216, A01H 5/00

(54) **Cooked potatoes**

(30) Priority: 01.11.1996 EP 96307938
(71) Applicant: UNILEVER PLC, London EC4P 4BQ (GB); UNILEVER N.V., 3013 AL Rotterdam (NL)
(72) Inventor: Gidley, Michael John, Sharnbrook, Bedford, MK44 1LQ (GB); Ormerod, Andrew Paul, Sharnbrook, Bedford, MK44 1LQ (GB)
(74) Representative: Kirsch, Susan Edith

(57) **Abstract**

A cooked potato having a yielding force of less than 60 N and containing potato cells having, on average, at least 5% of their intra-cellular volume occupied by non-starch aqueous matter. The cooked potato has a higher intra-cellular water content than normal cooked potatoes.

## Description

### FIELD OF THE INVENTION

The present invention relates to cooked potatoes and to products made therefrom.

### BACKGROUND ART

The potato is one of the major food crops in the world. Since they are produced in high yields and are relatively inexpensive, potatoes are the mainstay in the diet of people in many parts of the world. Normally the consumer either prepares and cooks raw potatoes or heats processed-potato products. Potatoes are commonly processed by, for example, boiling and mashing; chopping and frying; and baking.

Raw white potatoes comprise about 63 to 87 % water, 13 to 37 % total solids, 1 to 5 % protein, less than 1 % fat, 13 to 31 % carbohydrate and less than 2 % ash.

Starch, comprising about 65 to 80 % dry weight of potato, is calorifically the most important nutritional component. In raw potatoes, starch is present as microscopic granules in the potato cells. The granules are ellipsoidal in shape, and have a size of about 60 microns by 40 microns on average. The two main components of the starch are the polymers amylose and amylopectin.

When the temperature of a potato is raised to above about 50 degrees C, the water in the potato passes from the non-starchy parts of the cell into the starch granule, which then experiences limited swelling. At temperatures of 60 to 70 degrees C, the swollen starch granules undergo gelatinisation.

Starch gelatinisation has been defined (W A Atwell et al, Cereal Foods World 33, 306-11, 1988) as 'the collapse (disruption) of molecular orders within the starch granule manifested in irreversible changes in properties such as granular swelling, native crystallite melting, loss of birefringence, and starch solubilisation. The point of initial gelatinisation and the range over which it occurs is governed by starch concentration, method of observation, granule type, and heterogeneities within the granule population under observation.'

Gelatinisation may be determined by a number of methods, including Differential Scanning Calorimetry (DSC) which detects the temperature range and enthalpy associated with the collapse of molecular orders within the granule. To obtain accurate and meaningful results, the peak and/or onset temperature of the endotherm observed by DSC is usually determined.

A paper by Kim et al in the Journal of Food Science, Vol 60, No 5, 1995 , p 1060 - 1065, describes the screening of potato properties using DSC. Table 1 details the temperatures for the onset, peak and conclusion of starch gelatinisation for 42 potato genotypes. The peak temperatures are all in the range of 62 to 70 degrees C.

Following the gelatinisation process, potato starch granules swell extensively; this is generally known as pasting.

Pasting has been defined (W A Atwell et al, Cereal Foods World 33, 306-11, 1988) as 'the phenomenon following gelatinisation in the dissolution of starch. It involves granular swelling, exudation of molecular components from the granule, and eventually, total disruption of the granules.' Within a potato tuber, this swelling process typically results in the filling of intra-cellular volumes.

US 3669686 relates to a method of pretreating potatoes by heat tempering (annealing) them; during and after subsequent cooking the pretreated potatoes resist physical breakdown, remain firm and have the texture of partially raw potatoes. This firmness and texture are not obtained or desired in the cooked potatoes of the present invention.

WO 95/26407 discloses a method of producing altered starch from transformed potato plants; the potato tubers comprise an effective portion of a starch branching enzyme cDNA sequence operably linked in the antisense orientation to a suitable promoter; starch is extracted from these potatoes by homogenising them and washing and filtering the homogenate; the washed starch granules have an elevated temperature of gelatinisation and an elevated viscosity onset temperature. The cooking of these transformed potatoes is not disclosed or hinted at.

The filling of intra-cellular volume which occurs during pasting when ordinary potatoes are cooked results in a loss of free water within potato cells, such that the cooked potato has a dry consistency. If the cooked potato is subsequently processed by, for example, mashing, the gelatinised starch causes the mashed potato to be highly viscous. If the mashed potato is to be shaped (eg to form potato waffles), it is difficult to transport the potato mash through tubes from the mashing station to the shaping station, since its viscous nature means it has little or no flow. Moreover, the apparatus may become quickly clogged up with glutinous potato debris.

The present invention seeks to solve these problems by providing a cooked potato having a greater content of free water, thereby enabling more convenient processing of the potato, as well as providing cooked potatoes and products made therefrom having a more succulent mouthfeel.

### SUMMARY OF THE INVENTION

According to the present invention, there is provided a cooked potato having a yielding force of less than 60 N; wherein the potato comprises cells having, on average, at least 5% of their intra-cellular volume occupied by non-starch aqueous matter.

Preferably, the cooked potato has a yielding force of less than 50 N, more preferably less than 40 N.

The yielding force is the minimum amount of compressive force applied to a cylindrical piece of potato, which has a diameter of 1 cm and a height of 1 cm, that causes the potato piece to yield. The method used in the present invention is described below.

The yielding force is an important parameter as a cooked potato requires its tissue to have a minimum softness if it is to be either palatable or suitable for further processing. The softness of potato tissue is conveniently monitored by compressive mechanical tests which measure yielding forces.

Preferably, the potato cells have, on average, at least 10% of their intra-cellular volume occupied by non-starch aqueous matter, more preferably at least 20%.

The cooked potato of the present invention is preferably prepared by heating it at a temperature of at least 80 degrees C for a time sufficient to achieve a yielding force of less than 60N. For example, by heating it at 80 degrees C for 15 minutes, 100 degrees C for 5 minutes or alternative times and temperatures which result in an approximately equivalent heat input.

The present invention may be achieved by cooking a potato having a starch gelatinisation temperature of greater than or equal to 75 degrees C. It is also achievable by cooking a potato having a starch gelatinisation temperature of less than 75 degrees C and having limited post-gelatinisation starch swelling.

For the purpose of the present invention, the starch gelatinisation temperature is defined as the peak temperature of a DSC endotherm.

Potatoes having a starch gelatinisation temperature of greater than or equal to 75 degrees C are disclosed in WO 96/34968 (International patent application no PCT/GB96/01075) . The contents of WO 96/34968 are incorporated herein by reference thereto.

The potatoes disclosed in WO 96/34968 have characteristics which are altered compared to normal potatoes; this is as a consequence of introducing into normal potatoes a portion of a nucleotide sequence encoding an effective portion of a class A starch branching enzyme.

The altered characteristics include starch gelatinisation temperatures which are higher than those for normal potatoes, together with limited post-gelatinisation starch swelling.

Without wishing to be bound by theory, it is believed that the elevated starch gelatinisation temperature and limited post-gelatinisation starch swelling are a result of longer amylopectin polymer chains, and/or increased order in the lattice configuration of amylopectin polymers.

As a consequence of the elevated temperature of starch gelatinisation and/or limited post-gelatinisation starch swelling, potatoes can be heated to the extent that they are sufficiently softened for, eg, subsequent processing or eating, but not to the extent that complete starch gelatinisation and/or swelling of the starch granules takes place. Hence, the cells of the cooked potato are not completely full of swollen starch and the cooked potato product retains at least some of its free water; ie a significant portion of the intra-cellular volume is occupied by non-starch aqueous matter.

Alternative methods of providing a cooked potato having an elevated starch gelatinisation temperature and/or limited post-gelatinisation starch swelling include annealing, in which the annealing conditions are selected so as to alter the physical state of a potato in such a way that it has the properties we claim. For example, a potato is heated for 15 to 48 hours at a temperature of just below (eg 5 °C below) its starch gelatiniation temperature.

In using such an annealing process, the molecular structure of the amylopectin polymers is not altered but their ordered configuration is made more stable, resulting in an increase in the temperature of starch gelatinisation and/or a reduction in post-gelatinisation starch swelling.

### DETAILED DESCRIPTION OF THE INVENTION

Examples of the products and processes of the invention will now be described to illustrate, but not to limit, the invention, with reference to the accompanying figures, in which:
figure 1 is a micrograph of a prior art raw potato of the Desiree variety,
figure 2 is a micrograph of a prior art potato of the Desiree variety after heating at 70°C for 5 minutes,
figure 3 is a force/displacement trace of a prior art potato of the Desiree variety after heating at 70°C for 5 minutes,
figure 4 is a force/displacement trace of a prior art potato of the Desiree variety after heating at 80°C for 15 minutes,
figure 5 is a micrograph of a potato of the present invention after heating at 100°C for 5 minutes,
figure 6 is a micrograph of a potato of the present invention after heating at 80°C for 15 minutes,
figure 7 is a micrograph of a prior art potato chip,
figure 8 is a micrograph of a potato chip of the present invention.

### Comparative Example A

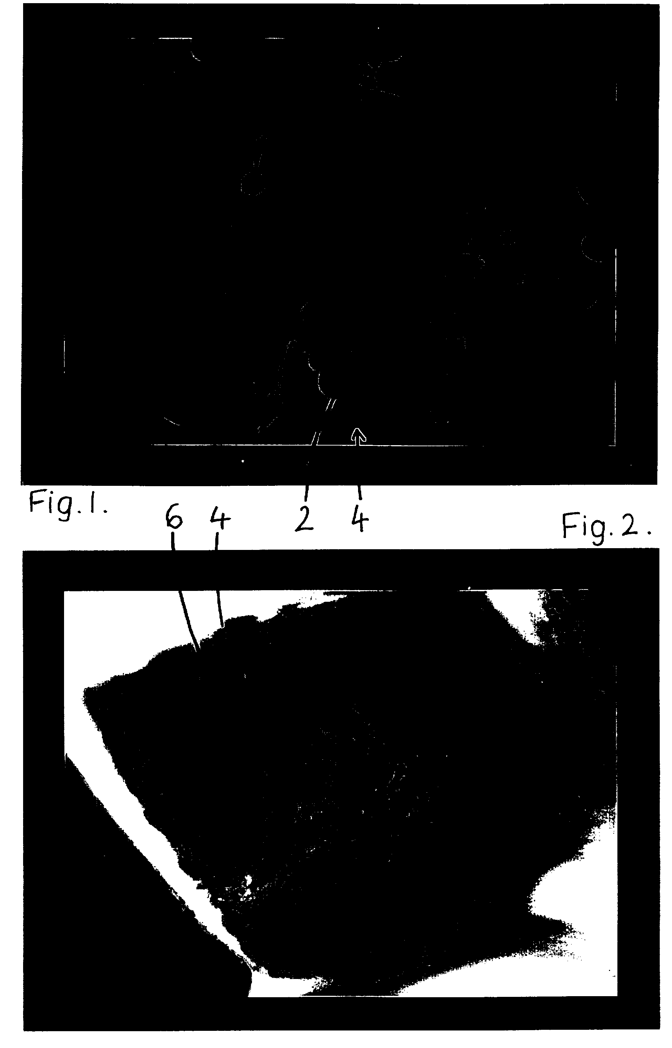

Using light microscopy, a micrograph was taken of a slice of a raw Desiree potato, available from supermarkets in the UK, that had been stained with Iodine. Figure 1 shows the unswollen starch granules 2 in cells 4 of the potato slice.

A cylindrical piece of raw Desiree potato was heated in water at a temperature of 70 degrees C for 5 minutes. A micrograph was taken of a slice of the cooked potato piece stained with iodine. Figure 2 shows the resulting swollen starch granules 6 in cells 4 of the potato slice.

### Results

Figures 1 and 2 illustrate that, upon heating, the starch granules of a common potato variety gelatinise and swell, such that substantially all the intra-cellular volume is occupied by swollen starch granules. There is negligible intra-cellular non-starch aqueous matter as all free water in the raw potato cell has been absorbed by the starch granules during gelatinisation and swelling. Sometimes the cells rupture as a result of the pressure exerted by the swollen starch granules.

### Comparative Example B

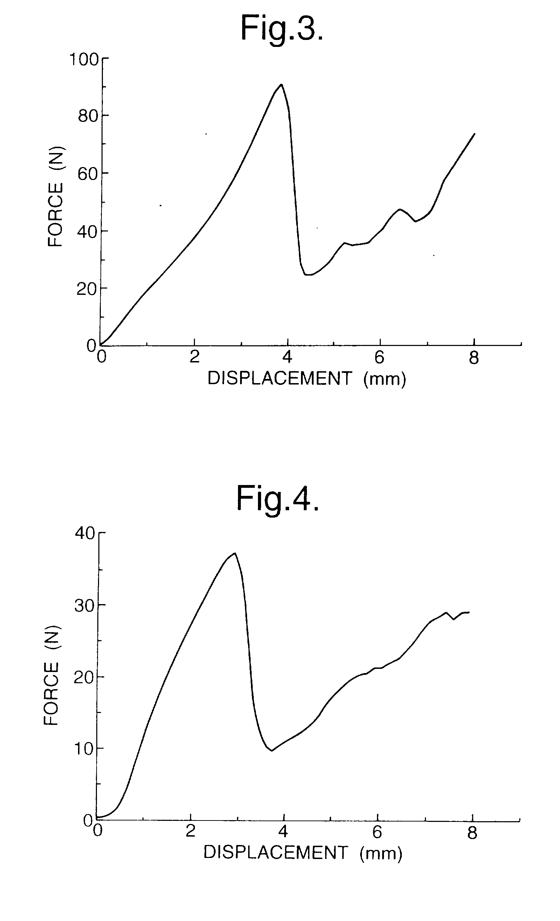

A cylindrical piece of raw Desiree potato was heated in water at a temperature of 70 degrees C for 5 minutes. The piece of the potato, measuring 1 cm in diameter and 1 cm in height, was compressed in a Universal Testing Machine, model 4501, available from Instron, at a strain rate of 100 mm/minute. A force/displacement trace was obtained and is shown in figure 3.

### Results

The cooked potato piece had a peak yielding force of 90 - 100 N, which is too high for either traditional food use or further processing such as mashing.

### Comparative Example C

The method of comparative example B was repeated, except that the piece of raw Desiree potato was heated in water at a temperature of 80 degrees C for 15 minutes. The force/displacement trace is shown in figure 4.

### Results

The cooked potato had a peak yielding force of 35 - 40 N, which is indicative of the potato tissue being sufficiently soft for either traditional food use or further processing.

### Example 1

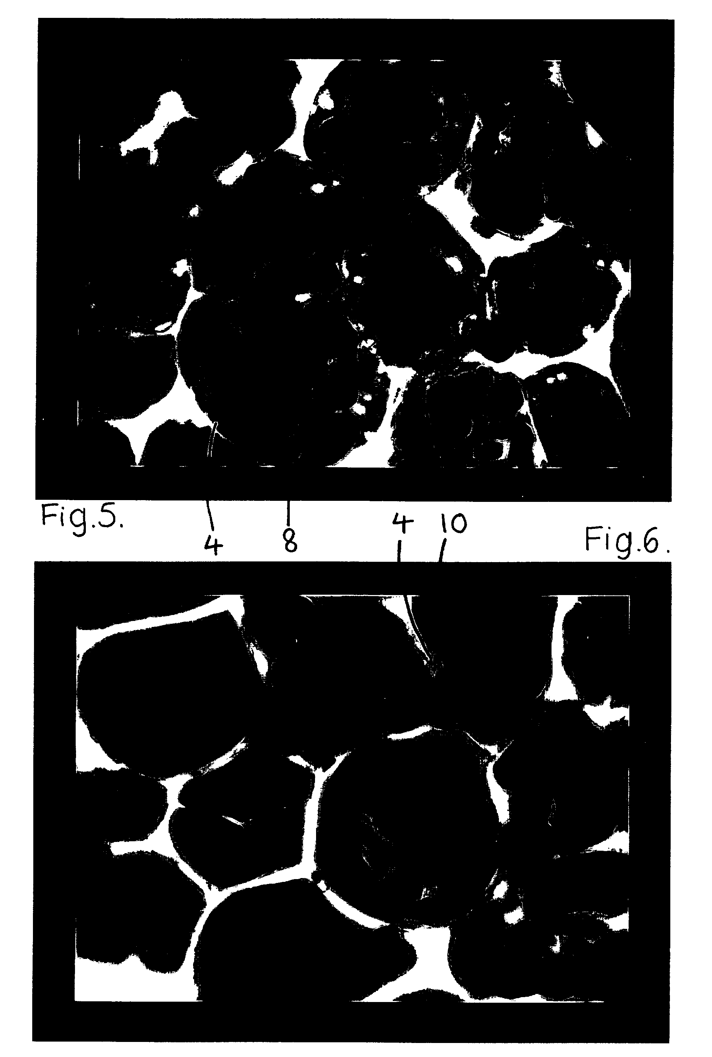

Cylindrical pieces of raw potato of a genotype disclosed in WO 96/34968 (sample 208 which is described as AS-Class B SBE(15) + AS-Class A SBE) were heated in water at a temperature of 100 degrees C for 5 minutes. A micrograph was taken of a slice of a cooked potato piece which had been stained with iodine. Figure 5 shows the resulting starch granules 8 in cells 4 of the potato slice. A force/displacement trace was obtained of a cylindrical potato piece using the method described in comparative example B.

On the assumption that the intra-cellular volume of potato cells is occupied only by starch granules and by non-starch aqueous matter, the volume occupied by the non-starch aqueous matter is calculated from micrographs by assessing the ratio of starch granules to non-starch aqueous matter from a cross-sectional view of the cell.

### Results

The cooked potato had a peak yielding force of about 10 N, which is indicative of the potato tissue being sufficiently soft for either traditional food use or further processing. Figure 5 illustrates that the starch granules were swollen to a limited degree, such that about 43% of the intra-cellular volume was occupied by non-starch aqueous matter.

### Example 2

The method of example 1 was repeated, except that the potato piece was heated at a temperature of 80 degrees C for 15 minutes.

### Results

The cooked potato had a peak yielding force of about 40 N, which is indicative of the potato tissue being sufficiently soft for either traditional food use or further processing. The starch granules were swollen to a limited degree, such that about 30% of the intra-cellular volume was occupied by non-starch aqueous matter.

### Example 3

Cylindrical pieces of potato of the Desiree variety were heated in water at a temperature of 55 degrees C for 24 hours and then at 60 degrees C for 15 hours. These temperatures were selected as they are just below the starch gelatinisation temperatures. Long heating times were used to effect annealing. The potato pieces were then heated at 80 degrees C for 15 minutes to cook them. A micrograph was taken of a slice of a cooked potato piece that had been stained with iodine. Figure 6 shows the resulting starch granules 10 in cells 4 of the potato slice.

### Results

The cooked potato was sufficiently soft for either traditional food use or further processing (ie it had a yielding force of less than 60 N). The starch granules were swollen to a limited degree, such that about 10% of the intra-cellular volume was occupied by non-starch aqueous matter.

Examples 4 to 7 below describe a range of application tests carried out on prior art potatoes of the Desiree variety (referred to as the control potato) and potatoes of the genotype used in Example 1 (referred to as the transgenic potato).

### Example 4

Control and transgenic potatoes were cut into pieces of about 125 cm³ and placed in a pressure cooker containing about 250 ml of cold tap water. They were cooked for 15 minutes at full pressure.

### Results

The control potato had a dry texture when cut, whereas the transgenic potato had a moist and succulent texture.

### Example 5

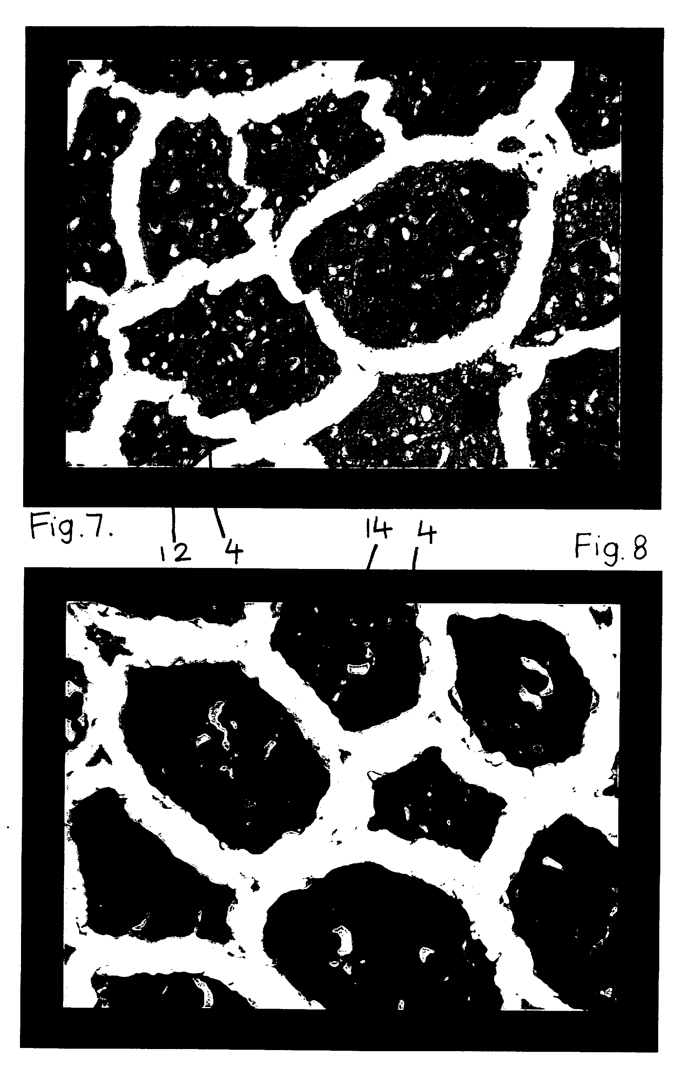

Control and transgenic potatoes were cut into chips (french fries) using a hand chipping machine. The chips were immersed in palm oil at 180 degrees C for 4 minutes. Micrographs were taken of a slice of a chip of each potato, after the slice had been stained with iodine. Figure 7 shows the resulting starch granules 12 in cells 4 of the slice taken from the control potato chip. Figure 8 shows the resulting starch granules 14 in cells 4 of the slice taken from the transgenic potato chip.

### Results

From figures 7 and 8, it can be seen that the cells of the transgenic potato chip contain a significant amount of non-starch aqueous matter (ie free water), in contrast to the cells of the control potato which contain negligible non-starch aqueous matter (ie almost no free water).

### Example 6

A microwave/convection oven was pre-heated to 200 degrees C for 5 minutes. Control and transgenic potatoes were cooked using high microwave power and a convection setting of 200 degrees C for 3 minutes per potato.

### Results

The control potato showed a characteristic dry texture on cutting, whereas the transgenic potato was juicy, as judged by the expression of water.

### Example 7

Control and transgenic potatoes were heated in water at a temperature of 80 degrees C for 15 minutes. The cooked potatoes were mashed.

### Results

After heating, the potatoes were sufficiently soft for either traditional food use or for subsequent processing (ie they had a yielding force of less than 60 N). After mashing, the control potato was sticky and glutinous, whereas the transgenic potato was moist and non-glutinous.

Cooked potatoes of the present invention may be in the form of mashed potato, chips, crisps, waffles, baked potatoes or any other form resulting from the many traditional preparations of potato for food use. The potatoes may be cooked by boiling, frying, grilling or baking.

## Claims

1. Cooked potato having a yielding force of less than 60 N; wherein the potato comprises cells having, on average, at least 5% of their intra-cellular volume occupied by non-starch aqueous matter.

2. Cooked potato as claimed in claim 1, wherein at least 10% of the intra-cellular volume of the potato cells is occupied by non-starch aqueous matter.

3. Cooked potato as claimed in claim 2, wherein at least 20% of the intra-cellular volume of the potato cells is occupied by non-starch aqueous matter.

4. Cooked potato as claimed in any preceding claim, wherein the potato is cooked by heating it at a temperature of at least 80 degrees C.

5. Cooked potato as claimed in any preceding claim, having a yielding force of less than 50 N.

6. Cooked potato as claimed in claim 5, having a yielding force of less than 40 N.

7. Cooked potato as claimed in any preceding claim, having a starch gelatinisation temperature of greater than or equal to 75 degrees C.

8. Cooked potato as claimed in any preceding claim, being a cooked transgenic potato.

9. Cooked potato as claimed in claim 8, either being a potato having characteristics altered by introducing therein a nucleotide sequence encoding an effective portion of a class A starch branching enzyme obtainable from potato plants or being the progeny of the potato having said altered characteristics.

10. Cooked potato as claimed in any one of claims 1 to 7, being a potato having characteristics altered by physical means.

11. Cooked potato as claimed in claim 10, wherein the physical means is annealing.

12. Cooked potato as claimed in any preceding claim, wherein the potato is cooked by boiling, frying, grilling or baking.
